(19)

Europäisches Patentamt
European Patent Office
Office européen des brevets

(11) **EP 3 963 308 B1**

(12) **EUROPEAN PATENT SPECIFICATION**

(45) Date of publication and mention
of the grant of the patent:
**19.02.2025 Bulletin 2025/08**

(21) Application number: **20822620.9**

(22) Date of filing: **11.06.2020**

(51) International Patent Classification (IPC):
**G01N 21/33** (2006.01)    **G01N 21/64** (2006.01)
**G01N 33/18** (2006.01)

(52) Cooperative Patent Classification (CPC):
**G01N 21/6486; G01N 21/33;** G01N 21/05;
G01N 33/182; G01N 33/1893; G01N 2021/1736;
G01N 2021/646; G01N 2021/6482

(86) International application number:
**PCT/IL2020/050645**

(87) International publication number:
**WO 2020/250226 (17.12.2020 Gazette 2020/51)**

(54) **SYSTEM AND METHOD FOR DETERMINING AQUEOUS NITRATE CONCENTRATION IN SOLUTION CONTAINING DISSOLVED ORGANIC CARBON**

SYSTEM UND VERFAHREN ZUR BESTIMMUNG DER WÄSSRIGEN NITRATKONZENTRATION IN EINER LÖSUNG MIT GELÖSTEM ORGANISCHEN KOHLENSTOFF

SYSTÈME ET PROCÉDÉ DE DÉTERMINATION D'UNE CONCENTRATION AQUEUSE DE NITRATES DANS UNE SOLUTION CONTENANT DU CARBONE ORGANIQUE DISSOUS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.06.2019 US 201962860273 P**

(43) Date of publication of application:
**09.03.2022 Bulletin 2022/10**

(73) Proprietor: **B.G. Negev Technologies & Applications Ltd.,
at Ben-Gurion University
8410501 Beer-Sheva (IL)**

(72) Inventors:
• **ARNON, Shlomi**
**8533800 Lehavim (IL)**
• **DAHAN, Ofer**
**8499000 Sde Boker (IL)**
• **YESHNO, Elad**
**8499000 Sde Boker (IL)**

(74) Representative: **Wright, Howard Hugh Burnby
Withers & Rogers LLP
2 London Bridge
London SE1 9RA (GB)**

(56) References cited:
WO-A1-2017/156258    WO-A1-2017/156258
WO-A1-2018/104939    CN-A- 109 655 110
CN-A- 109 655 110    US-A1- 2020 209 208

• **B.D. SHAW ET AL: "Analysis of Ion and Dissolved Organic Carbon Interference on Soil Solution Nitrate Concentration Measurements Using Ultraviolet Absorption Spectroscopy",** VADOSE ZONE JOURNAL, vol. 13, no. 12, 19 December 2014 (2014-12-19), pages 1 - 9, XP055609478, DOI: 10.2136/vzj2014.06.0071
• **ANTHONY C. EDWARDS ET AL: "Determination of Nitrate in Water Containing Dissolved Organic Carbon by Ultraviolet Spectroscopy",** INTERNATIONAL JOURNAL OF ENVIRONMENTAL ANALYTICAL CHEMISTRY, vol. 80, no. 1, 2001, GB, pages 49 - 59, XP055672082, ISSN: 0306-7319, DOI: 10.1080/03067310108044385

EP 3 963 308 B1

## Description

### Field of the Invention

The invention relates in general to systems and methods for optimizing agricultural crops' yields while reducing water contamination due to excess application of fertilizers. More specifically, the invention relates to a system and method for determining a level of nitrate in a water sample, thereby to determine accurate amount of fertilization necessary.

### Background of the Invention

Contamination of rivers, lakes, fresh water, drinking water, groundwater and soil's porewater by nitrate is a global problem. The term "nitrate" is briefly referred to herein also as "N". It is universally recognized that nitrate contamination of drinking water is a threat to human health. It is also reported that human health suffers from adverse effects, even cancer, due to continual exposure to nitrate above a certain level. A significant rate of water pollution results from an excess of fertilization by farmers, due to a lack of real-time and accurate information with respect to the availability of nutrient in the soil. Therefore, an excess of fertilization results not only in a waste of resources, but also with a pollution of the groundwater, particularly by nitrate.

During the second half of the 20th century, clear trends of rising nitrate concentration in groundwater have been observed in aquifers all over the globe. The World Health Organization (WHO) had determined that nitrate levels in drinking water should not exceed 50ppm. When exceeding this level of concentration, nitrate is harmful to infants, where it may cause "blue-babies syndrome" (methemoglobinemia) and can lead to severe illness and even death. Unfortunately, nitrate contamination is the most dominant factor responsible to severe degradation of groundwater and surface resources. On a global scale, eutrophication and hypoxia of streams, rivers and lakes, is mostly attributed to subsurface return flow from nitrate contaminated groundwater, leaking from phreatic aquifers underlying agricultural fields. Moreover, the impact of nitrate contaminated groundwater is not limited to terrestrial water resources, and has a great impact on marine ecosystems as well. Eutrophication and hypoxia on a large scale has been found in the Gulf of Mexico and the Black Sea, as well as severeimpact on the Great Barrier Reef, Australia. Overall, nitrate contamination had led to more groundwater disqualification and water well shutdowns than any other contaminant, worldwide. While nitrate is considered the most common non-point source pollutant in groundwater, numerous studies have linked the increase of nitrate concentration in groundwater to excess use of fertilizers in agriculture. As a result, a global regulatory takes place by environmental protection and water authorities to reduce excessive application of agricultural fertilizers. For example, the European Union had established the Nitrates Directive, and the US Environmental Protection Agency (EPA) regards nitrate contamination in groundwater as an event requiring immediate action.

At present, fertilizer application in agriculture relies primarily on farmer's experience, expert's recommendation, and sporadic soil testing. None of these techniques provide information that is in line with the time scale of N-fertilizers mobilization (nitrate's solution movement rates through the soils sediments), consumption and transformation dynamics in the soil.

Presently, the monitoring of chemical parameters in soils is performed in water samples, that may be obtained, for example, by a suction cup which is mounted in the soil, or by extracting soil samples. Water samples collected by this mechanism is typically transferred to a laboratory for further chemical analysis, or is analyzed on-site by means of an analytical kit. However, nitrate in the soil is highly soluble, mobile, and is obviously consumed by the crops. Moreover, nitrate concentration in the soil may fluctuate in time-scales of hours to days, as a response to different irrigation schemes, precipitation, fertilization, root uptake and different plant growth phases. As such, the monitoring of nitrate concentration by conventional tools does not meet the required time resolution for optimizing fertilization schemes, while preventing groundwater pollution due to excess application of fertilizers. Moreover, current techniques typically require handling of the sampling schemes by a devoted research team, not by farmers.

Ultraviolet (UV) absorption spectroscopy is one of the most common methods for nitrate analysis in aqueous solution. Nitrate in aqueous solution absorbs light at two main wavelength regions: (a) a high-absorbance band in between 200-240nm; and (b) a low absorbance-band in between 280-320nm. While the absorbance in both bands is known to have direct correlation to nitrate concentration, the absorbance at 220nm is two orders of magnitude higher when compared to the absorbance at 300nm.

Tuly et al., (2009), "In Situ Monitoring of Soil Solution Nitrate: Proof of Concept", https://www.researchgate. net/publication/231523625, suggests a technique for continuous monitoring of nitrate concentrations in soil solution. An absorbance spectroscopy is applied on a sample within a stainless-steel porous cup, which is installed in the soil. The porous cup is filled with deionized water, which is placed in a reservoir of potassium nitrate solution. Once the solution inside of the cup achieves chemical equilibrium by diffusion between the porous cup and the surrounding medium, the absorption spectrum of the solution is measured by means of a UV dip probe. The proposed setup of Tuly et al, (2009) uses a UV light source and a dip probe that are connected to spectrophotometer via optical fibers, to continuously determine the nitrate concentration within the cup. However, this technique has a limited applicability, for two main reasons: (a) the obtaining of chemical equilibrium between the porous cup and the surrounding medium, especially in unsaturated sediment with a limited water storage, is rather slow, resulting in a time lag between the actual variation of the nitrate concentration in the soil to its actual measurement. Therefore, rapid concentration variations, as expected following intensive irrigation or fertilization events may not be recorded; and (b) the presence of natural soil Dissolved Organic Carbon (DOC) limit the accuracy of UV absorption by means of spectroscopy analysis, since both nitrate and soil DOC absorb UV light in overlapping wavelengths ranges.

WO 2018/104939 A1, Yeshno et al., 2019 suggests a nitrate monitoring technique which is based on a continuous spectral analysis of soil porewater in an optical flow cell. The optical flow cell is connected to a porous interface which obtains a continuous flux of soil porewater. The absorption spectrum of the soil porewater is continuously recorded and analyzed to determine in real-time the nitrate concentration. The analysis involves a scan of the absorption spectrum of the soil porewater to identify an optimal wavelength where DOC interference to nitrate measurement is minimal. The system for carrying out this technique requires for its operation a wide-band UV spectrophotometer and accordingly a wide-range, deuterium UV lamp. However, in spite of the capability of the system to continuously measure nitrate concentration *in-situ,* the system's large- dimensions, along with its high energy consumption, is too bulky and expensive for practical and commercial applications.

In brief, the prior art optical system of WO 2018/104939 A1 for determining a level of nitrate in a cultivated soil basically requires the following components:

a. One or more sampling cells, each enabling the extraction of a porewater sample from a specific region of the soil;
b. Each sampling cell is connected to an optical flow cell for continuous or frequent spectral analysis of the soil porewater;
c. A UV light source for applying a light beam in wavelengths between 200-240nm through the water sample in the flow cell;
d. A photodetector or a spectrophotometer for accumulating light from the light beam, following its passage through the water sample;
e. A processing unit for determining the level of light-absorption (based on Beer-Lambert equation), and
f. A processing unit to estimate nitrate concentrations based on a previously prepared empiric calibration equation. The calibration equation is obtained by an algorithm which locates an optimal wavelength, where a minimal interference from DOC is found;
a. WO 2018/104939 A1 also shows how the system can perform analyses on a plurality of regions, while each time a single region is selected for analyses. It is an object of the present invention to provide a system for determining in real time and *in-situ* a level of nitrate in soil, which is simpler in structure, compact in size, and of lower cost compared to similar prior art systems.

It is a particular object of the invention to eliminate the effects of DOC existing in a porewater sample on the absorption spectrum, thus to enable the use of absorption spectroscopy technique to estimate aqueous nitrate concentration in the presence of DOC in the solution.

Other objects and advantages of the invention will become clear as the description proceeds.

CN-A-109655110 suggests a composite packaged LED light source and a multiparameter water quality monitoring device based on the light source, such that so that nitrate, turbidity and chromaticity indexes can be simultaneously measured.

WO-A1-2017/156258 suggests an optical nitrate sensor for determining the quality of water including multiparameter water quality monitoring.

B.D. SHAW ET AL, in "Analysis of Ion and Dissolved Organic Carbon Interference on Soil Solution Nitrate Concentration Measurements Using Ultraviolet Absorption Spectroscopy", VADOSE ZONE JOURNAL, vol. 13, no. 12, 19 December 2014, pages 1-9, DOI: 10.2136/vzj2014.06.0071 suggests an in situ nitrate monitoring probe and examines its performance in the presence of potentially interfering ionic species and dissolved organic carbon (DOC).

ANTHONY C. EDWARDS ET AL, in "Determination of Nitrate in Water Containing Dissolved Organic Carbon by Ultraviolet Spectroscopy", International Journal of Environmental Analytical Chemistry, vol. 80, no. 1, 2001, pages 49-59, GB, ISSN: 0306-7319, DOI: 10.1080/03067310108044385 suggests a two-wavelength approach for the correction of absorbance that organic matter, such as nitrate might have contributed.

## Summary of the Invention

The invention relates to a system for determining a level of nitrate in a water sample as defined in claim 1. The system comprises:
(a) an optical flow cell which is at least partially transparent and which is configured to contain a sample of water; (b) a first illuminator for illuminating the sample within the cell by light in a first wavelength, and a first photodetector for collecting the first-wavelength illumination, following the light passage through the sample; (c) a second illuminator for illuminating the sample within the cell by light in a second, fluorescence-excite wavelength, and a second photodetector for collecting illumination in a third, fluorescence-emission wavelength from the sample; and (d) an analysis unit for: (d.1) determining an overall effect of nitrate+DOC (hereinafter, the terms "nitrate+DOC" and "nitrate and DOC" are used interchangeably, and their essence is the same) within the sample on the absorbance of the sample, said effect of nitrate+DOC being proportional to a rate of absorbance of light due to said illumination by said first illuminator, said absorbance being determined from a difference between a level of illumination by said first illuminator and a level of collected illumination by said first photodetector; (d.2) determining a concentration of DOC within the sample, said DOC concentration being proportional to an intensity of said fluorescence emission from the sample due to said illumination by said second illuminator, and as collected by said second photodetector; and (d.3) subtracting said effect of DOC from said effect of nitrate+DOC on absorbance, thereby to determine the concentration of nitrate within the sample.

In an embodiment of the invention, the system further comprising a first look-up table, for converting the difference as measured in step (d.1) to a nitrate and DOC concentration level.

In an embodiment of the invention, the system further comprising a second look-up table for converting said fluorescence emission as measured in step (d.2) to a DOC concentration level.

In an embodiment of the invention, the system further comprising a third look-up table, for calibrating the subtraction result based on a specific type of DOC known to be in the specific tested sample, wherein said type of DOC reflects a specific chemical DOC composition.

In an embodiment of the invention, a mathematical equation is used to convert absorbance and/or fluorescence measurements to concentration levels.

In an embodiment of the invention, the system further comprising one or more additional illuminators, and one or more additional photodetectors, in order to measure absorbance and/or fluorescence emission in additional wavelengths, thereby to determine a specific type of DOC within the sample.

In an embodiment of the invention, the analysis unit comprises a mathematical model to extract the value of nitrate based on the measurements of absorption and fluorescence, wherein the mathematical model comprising:

$$C_{NO_3^{-1}} = \frac{C_{DOC}\left(\frac{A_{NO_3^{-1}}(\lambda_1)+A_{DOC}(\lambda_1)}{A_{NO_3^{-1}}(\lambda_2)+A_{DOC}(\lambda_2)}\right)x\varepsilon_{DOC}(\lambda_2)-\varepsilon_{DOC}(\lambda_1)}{\varepsilon_{NO_3^{-1}}(\lambda_1)-\left(\frac{A_{NO_3^{-1}}(\lambda_1)+A_{DOC}(\lambda_1)}{A_{NO_3^{-1}}(\lambda_2)+A_{DOC}(\lambda_2)}\right)x\varepsilon_{NO_3^{-1}}(\lambda_2)} \qquad (3)$$

Where A is the measured absorbance at a given wavelength ($\lambda_{1,2}$(nm)), $\varepsilon_{NO_3^{-1},DOC}\left(\lambda_{1,2}(nm)\right)$ is a molar attenuation coefficient for either the nitrate or the DOC (L mol$^{-1}$ cm$^{-1}$) at a given wavelength ($\lambda_{1,2}$(nm)), $C_{DOC}$ is the DOC concentration (mol L$^{-1}$) as obtained from said second photodetector, $C_{NO_3}$ is a nitrate concentration (mol L$^{-1}$), and $l$ is an optical pathlength (cm).

In an embodiment of the invention, the analysis unit applies a machine learning technique comprising: (a) generating a plurality of absorption and fluorescence measurements for different values of nitrate concentration and various DOC types and respective concentrations; (b) selecting and adapting one or more deep learning networks; (c) training at least one of the selected deep learning networks; and (d) using the trained network to calculate the nitrate concentration based on absorption and fluorescence measurements.

In an embodiment of the invention, the first wavelength is selected from the bands of 200-250nm and 280-320nm.

In an embodiment of the invention, the second excite wavelength is within a band of 225nm-600nm.

In an embodiment of the invention, the third, fluorescence emission wavelength is within a band of 250nm-700nm.

In an embodiment of the invention, the system further comprising a first filter for assuring that radiation only in the first wavelength arrives the first photodetector.

In an embodiment of the invention, the system further comprising a second filter for assuring that radiation only in the third wavelength arrives the second photodetector.

In an embodiment of the invention, the water sample is taken from a soil or from a water reservoir.

In an embodiment of the invention, the water sample is collected from a cultivated soil by a porous interface, and is provided in a low flow-rate through the optical flow cell.

The invention further relates to a method for determining a concentration rate of nitrate in a water sample as defined in claim 10. The method comprises:
(a) providing the sample; (b) illuminating the sample in a first wavelength, and determining the combined effect of nitrate+DOC within the sample on the absorbance, said concentration of nitrate+DOC being proportional to a rate of absorbance of light due to said illumination in said first wavelength, said absorbance being determined from a difference between a level of illumination in said first wavelength before passing the sample and a level of collected illumination in said first wavelength following passage through the water sample; (c) illuminating the water sample in a second, exciting wavelength, and determining an effect of DOC within the sample, said effect of DOC being proportional to an intensity of fluorescence emission from the sample in a third wavelength due to said illumination of the sample in said second wavelength; and (d) deducting the effect of DOC from the overall effect of nitrate+DOC on the absorbance, as determined, thereby to obtain the concentration of nitrate in the sample.

In an embodiment of the invention, the method further comprising use of a first look-up table for converting the absorbance to a nitrate+DOC concentration level.

In an embodiment of the invention, the method further comprising use of a second look-up table for converting said fluorescence emission to a DOC concentration level.

In an embodiment of the invention, the method further comprising use of a third look-up table, for calibrating the deduction result based on a specific type of DOC known to be in the specific tested sample, wherein said type of DOC reflects a specific chemical DOC composition.

In an embodiment of the invention, the method further comprising use of a mathematical equation to convert absorbance and/or fluorescence measurements to concentration levels.

In an embodiment of the invention, the method further comprising measuring absorbance and/or fluorescence emission in additional wavelengths, thereby to determine a specific type of DOC within the sample.

In an embodiment of the invention, the method comprising a mathematical model to extract the value of nitrate based on the measurements of absorption and fluorescence, wherein the mathematical model comprising:

$$C_{NO_3^{-1}} = \frac{C_{DOC}\left(\frac{A_{NO_3^{-1}}(\lambda_1)+A_{DOC}(\lambda_1)}{A_{NO_3^{-1}}(\lambda_2)+A_{DOC}(\lambda_2)}\right)x\varepsilon_{DOC}(\lambda_2)-\varepsilon_{DOC}(\lambda_1)}{\varepsilon_{NO_3^{-1}}(\lambda_1)-\left(\frac{A_{NO_3^{-1}}(\lambda_1)+A_{DOC}(\lambda_1)}{A_{NO_3^{-1}}(\lambda_2)+A_{DOC}(\lambda_2)}\right)x\varepsilon_{NO_3^{-1}}(\lambda_2)} \qquad (3)$$

Where $A$ is the measured absorbance at a given wavelength ($\lambda_{1,2}$(nm)), $\varepsilon_{NO_3^{-1},DOC}\left(\lambda_{1,2}(nm)\right)$ is a molar attenuation coefficient for either the nitrate or the DOC (L mol$^{-1}$ cm$^{-1}$) at a given wavelength ($\lambda_{1,2}$(nm)), $C_{DOC}$ is the DOC concentration (mol L$^{-1}$) as obtained from said second photodetector, $C_{NO_3}$ is a nitrate concentration (mol L$^{-1}$), and $l$ is an optical pathlength (cm).

In an embodiment of the invention, the first wavelength is selected from the bands of 200-250nm, or 280-320nm.

In an embodiment of the invention, the second, exciting wavelength is in the order of 225 - 400 nm.

In an embodiment of the invention, the third, fluorescence emission wavelength is in the order of 250nm-500nm.

In an embodiment of the invention, the water sample is taken from a soil or from a water reservoir.

In an embodiment of the invention, the method further applies a machine learning technique, comprising the steps of (a) generating a plurality of absorption and fluorescence measurements for different values of nitrate concentration and different types of DOC, and their respective DOC concentrations; (b) selecting one or more deep learning networks; (c) training the network and selecting a one with a best performance; and (d) calculating the nitrate concentration based on said absorption and fluorescence measurements.

## Brief Description of the Drawings

In the drawings:

- Fig. 1 generally illustrates in a flow diagram form a method for determining a concentration of nitrate in a water sample, according to an embodiment of the invention;
- Fig. 2 schematically illustrates a structure of a system for determining a concentration of nitrate in soil porewater, according to an embodiment of the invention;
- Fig. 3 illustrates a method for determining nitrate concentration in soil solution (or aqueous solution), according to an embodiment of the invention;
- Fig. 4 exemplifies a complex pattern in the relationship between nitrate concentration, DOC concentration and a UV absorption for soil water as obtained from conventional greenhouse soil water samples;
- Fig. 5 shows a 3D projection of the experimental results;
- Fig. 6 shows a calibration curve for a DOC concentration;

- Fig. 7 shows observed (known) nitrate concentration vs. predicted nitrate concentration by the new analytical concept of the invention, for soil water samples from 6 agricultural sites.
- Fig. 8 shows observed vs. predicted nitrate concentrations, for the hummus soil mixture water extract, when the calibration equation was obtained for absorption at 235nm.

## Detailed Description of Preferred Embodiments

The invention provides an optical system for determining a level of nitrate in a cultivated soil, which overcomes drawbacks of similar prior art systems. In brief:

a. The system can use low-cost and low-energy components, such as LED type light sources and semiconductor type photodetectors;

b. While prior art systems are based on absorption measurement only, the system of the invention measures: (i) absorption resulting from illumination by a first light source to measure a combined effect of both nitrate and DOC; and (ii) a florescence emission resulting from illumination by a second light source to measure the concentration of DOC, and cancel its effect on the absorption measurement by use of a signal processing algorithm. Such a dual-measurement technique enables separation of the effect of DOC from the combined effect of nitrate+DOC on the measurement, thereby the technique enables determination of the actual concentration of nitrate in the water sample. This structure and analysis eliminate the necessity to utilize a cumbersome system with a relatively high power and high-cost light-source, and the necessity to utilize a spectrometer in order to determine an optimal operational frequency in which the effect of DOC on the measurement is minimal;

c. The system applies algorithms to calculate the nitrate concentration;

d. The system applies one or more databases for calibrating the measurement. Optionally, several databases are used to accommodate for different types of soils (therefore different compositions of materials and optical properties within the DOC);

e. A second embodiment of the system of the invention applies a mathematical model of the DOC concentration as a function of fluorescence power and the light absorption as a function of nitrate+DOC concentration and solves an equation of two variables;

f. A third embodiment of the system of the invention applies many measurements to train a deep learning network, which is later used to calculate the nitrate concentration based on two measurements of absorption and fluorescence measurements.

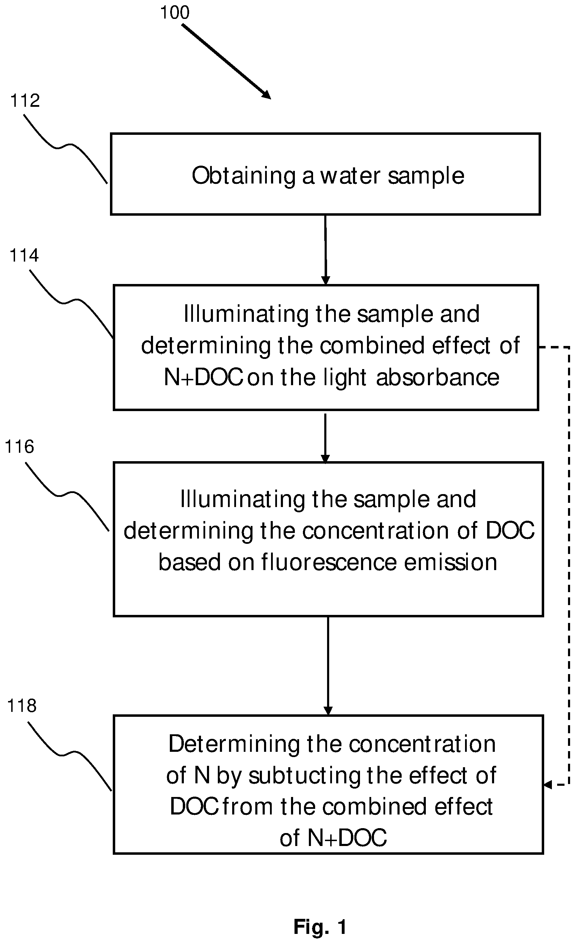

Fig. 1 generally illustrates in a flow diagram form a method 100 for determining a concentration of nitrate in a water sample (for example, which is taken from cultivated soil, waste water treatment plant effluent, hydroponic/aquaponic agriculture systems, rivers, lakes, and other agriculture-environmental applications. Hereinafter, for a sake of simplicity, the description will refer to a sample which is taken from soil) according to an embodiment of the invention. In step 112, a water sample is obtained from the soil, for example, by use of a porous interface. In one embodiment, the sample flows in a low flow-rate through an optical flow cell. In step 114, the water sample is illuminated, and the combined effect of the nitrate+DOC (referred to in the drawings as "N+DOC") on the absorbance is determined. In step 116, and following application of an excitation illumination on the sample in the optical flow cell, the concentration of DOC-only in the sample is determined by measuring the intensity of fluorescence emission from the sample. Finally, in step 118, the nitrate concentration in the sample is determined, by subtracting the effect of the DOC (as obtained in step 116) on the absorbance, from the combined absorbance at N+DOC concentration as obtained in step 114. The term "subtracting", which appears throughout this application does not necessarily refers to a pure mathematical operation, but rather to an operation which offsets the effect of DOC from the combination of N+DOC.

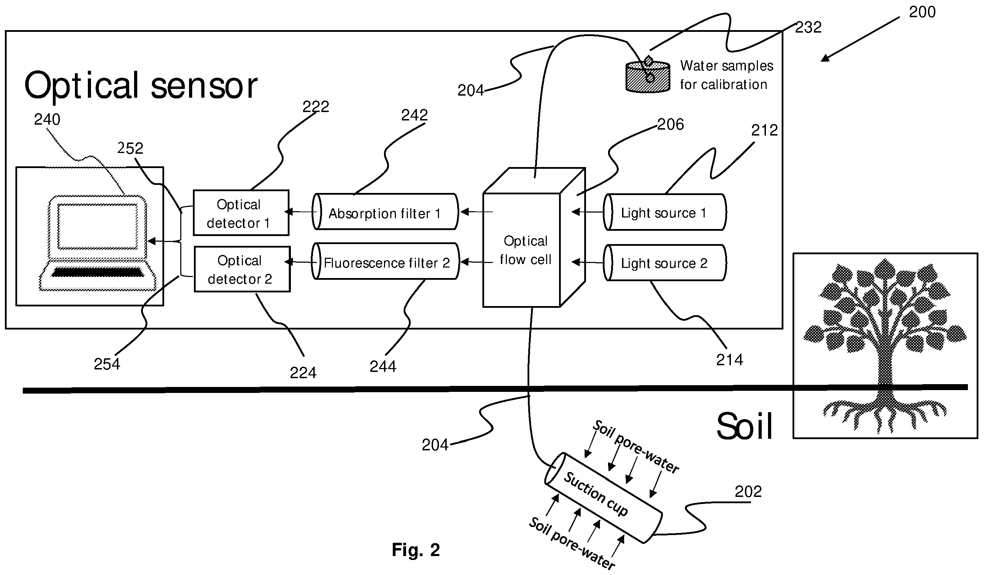

Fig. 2 schematically illustrates a structure of system 200 for determining a concentration of nitrate in soil porewater, according to an embodiment of the invention. A small dead-volume porous interface 202 is placed in the soil to obtain a continuous low flux stream of soil porewater solution. The soil porewater flows through an optical flow cell 206 via small diameter tubing (e.g., inner diameter ≤1.6 mm) 204. The sample extraction from the soil is driven, for example, by applying low pressure (vacuum) on the porous interface. The sample can be later discharged or accumulated for further analysis or system calibration at sample accumulation chamber 232. The casing of flow cell 206 is at least partially transparent, to allow passage of light beams therethrough. A first light source 212, preferably of LED type, illuminates the cell in a first UV wavelength, for example in a proximity of 300nm. The light beam of the first light source 212 passes through the optical flow cell 206 in which the soil porewater flows, while some of the light-beam's energy is absorbed by the water constituents (which in turn contains nitrate and DOC). The remaining energy from the light beam of the first light source 212 is accumulated by photodetector 222, forming an absorbance signal 252. A processing unit 240 is used to calculate the absorbance signal which reflects the difference between the illumination intensity by the first light source 212, and the light intensity which is accumulated by photodetector 222 (Beer Lambert equation). A second light source 214, preferably of a LED type, illuminates the cell 206 in a second wavelength, for example, in an excitation wavelength in proximity of 350nm. The light beam from the second light source 214 excites the DOC within the water sample, causing a DOC fluorescence emission at a secondary wavelength of, for example, 451nm. The fluorescence emission results substantially only from the DOC, is proportional to the DOC concentration, and is independent from the nitrate concentration within the sample. The fluorescence emission resulting from the second light beam is accumulated by second photodetector 224, forming a fluorescence signal 254. The fluorescence signal 254 is conveyed to the processing unit 240 where a predetermined calibration equation is used to estimate the DOC concentration in the sample. As previously mentioned, the fluorescence emission intensity is in proportion to the DOC concentration in the solution. However, the intensity of the fluorescence emission, in itself, is also proportional to the intensity of the excitation illumination by the second light source 214. Yet, the intensity of the excitation illumination is a parameter which is controlled by the operator of the system. Preferably, filters 242 and 244 are located in front of optical detectors 222 and 224, respectively, to ensure passage of only the wavelengths of interest towards the respective detectors. For example, a filter 244 allowing only light at 451nm is located in front of the fluorescence detector 224, ensuring that the 350nm light from the excitation beam will not be received and saturate the detector 224 or mask the fluorescence reading. In Addition, a filter for allowing only the passage of, for example, 300nm is located in front of the absorbance detector 222, to ensure that the measurement will only be carried at the region where nitrate has a maximum absorbance peak.

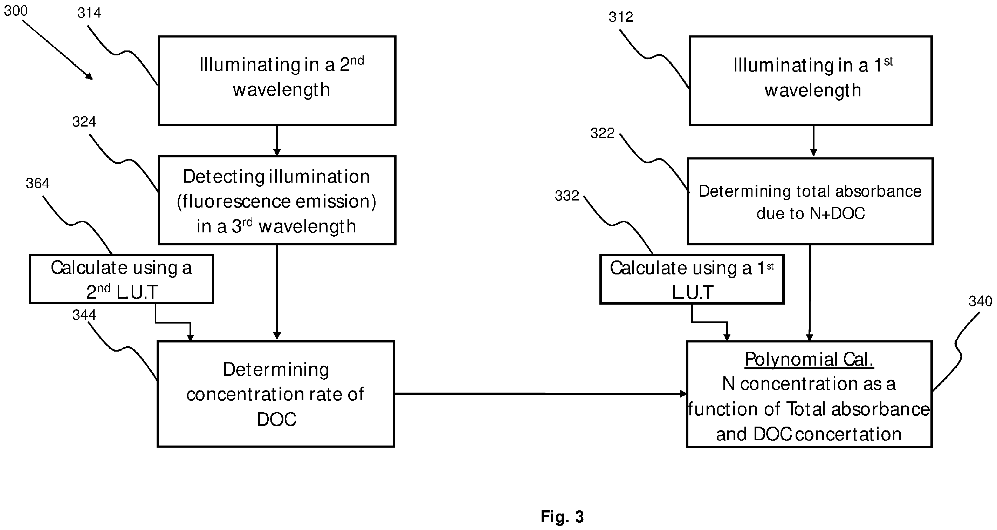

Fig. 3 illustrates a method 300 for determining nitrate concentration in soil (or in another medium containing nitrate, as discussed above), according to an embodiment of the invention. In step 312, the soil porewater is illuminated in a 1st wavelength by an illumination beam, typically either in the 200-240nm band, or preferably in the 280-320nm where a LED type illuminator can operate. In step 322, the total absorbance which is the outcome of the presence of both N+ DOC in the solution is calculated in reference to a predetermined blank solution, for example, double distilled water (DDW). In step 340 the calculated absorbance is substituted within a predetermined polynomial equation. This equation is further substituted with values from a 1st look-up-table 332 that provides a specific N / DOC concentration level for each specific combined absorption of N+DOC in the solution. Look-up-table 332 is mainly obtained through the use of experiments as will be later elaborated under the example section. Substantially simultaneously, the water sample is illuminated 314 by light in a 2nd wavelength, for example, 350nm or any other wavelength which is suitable to excite a fluorescence illumination of DOC from the sample. In step 324, a photodetector, which is tuned to detect in the wavelength of the fluorescence emission, for example 451nm, detects the intensity of the fluorescence emission from the sample. Fluorescence from excitation / emission at 350 / 451 nm results only from the existence of DOC in the sample. Moreover, it has been found that, for a given type of soil, the intensity of the fluorescence emission can be found in a linear or polynomial correlation with the concentration rate of DOC in the sample. In step 344, and given the intensity of the fluorescence emission as determined in step 324, the concentration of DOC in the sample is determined. This is also done by use of a calibration 2nd look-up-table 364. The calibration 2nd look-up-table 364, upon submission of a specific emission intensity, provides a specific concentration rate of DOC. The calibration look-up-table 364, is also prepared beforehand, typically by use of experiments. Preferably, look-up-table 364 selectively refers to the specific soil-type which is actually used. It has been found that the chemical composition of the DOC somewhat changes from one type of soil to another. Therefore, it is preferable to prepare a plurality of calibration look-up-tables 364, each for a specific type of soil, and to actually use the look-up-table (or data therefrom) which most suits the type of the examined soil. The DOC concentration as determined in step 344 is then substituted within the predetermined polynomial calibration equation 340. Finally, the polynomial equation uses the given DOC concentration to deduct its attribution to the combined absorption of N+DOC, as given by step 322. In other words, the polynomial equation returns the N concentration as a function of the DOC concentration (or its effect) in the solution, and the overall absorbance caused by N + DOC.

## Example

Soils from five different agricultural fields of the coastal plain of Israel, were collected and analyzed for this study, including: organic and conventional greenhouses for vegetable crops, open field which is used for rotating mixed crop, and a citrus's orchard. These sites were chosen to represent a spectrum of typical agricultural practices on different soils. In addition, commercial hummus soil mixture from "Dovrat" commercial compost was also examined to represent a potential impact of compost application in agriculture on the soil water DOC.

Soil solution samples were obtained from a soil and DDW mixture. The mixtures were left to stand for 24 hours to allow the solution to achieve a chemical equilibrium with the soil natural DOC. The soil phase and liquid phase in each sample was separated by a standard laboratory centrifuge, and the suspended solids were removed by 0.22pm membrane filters. The samples were then diluted to obtain a series of replicas reflecting different DOC concentrations. Each replica of DOC concentration was spiked with a specific volume of 10,000 ppm standard potassium nitrate solution, to obtain between 4 to 6 different nitrate concentrations per each level of DOC. As a result, a matrix composed of 25 to 30 samples of different combinations of DOC and nitrate concentrations, ranging from zero to about a 1000ppm nitrate, and between zero to about 100ppm DOC was created from the soil extracts at each agricultural site.

The initial values of DOC and Total Nitrogen (TN) in each sample were estimated by an *Analytic Jena multi N/C 2100s TOC/TN analyzer,* while the nitrate concentration in each sample was determined by *Dionex ICS 5000* Ion chromatograph. The absorption of each of the samples at 300nm was determined using *TECAN Spark 10M* multimode microplate reader spectrophotometer. The light absorbance was defined by the Lambert-Beer equation:

(1)

$$Absorbance = -log_{10}\frac{I}{I_0}$$

where *I* reflects the light intensity after passing through the examined solution and $I_0$ is the light intensity of the source, or the light intensity of the source after passing through DDW as a reference.

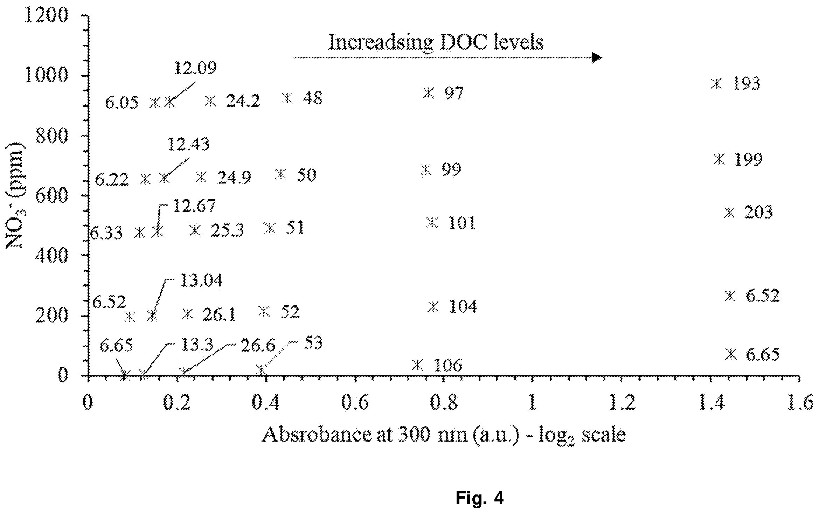

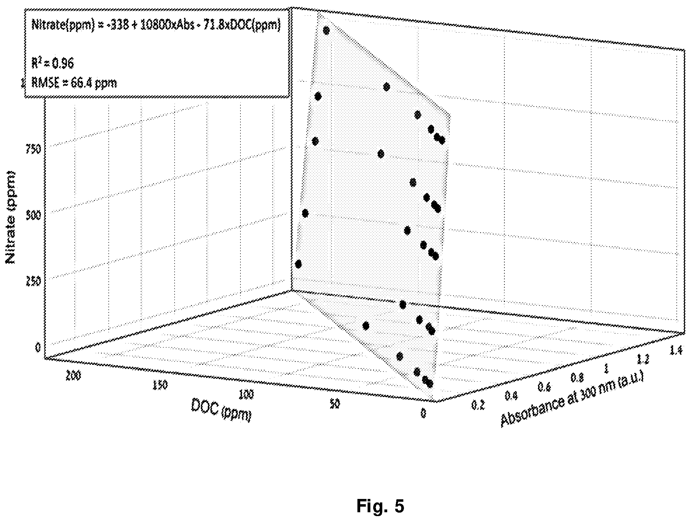

A fluorescence spectroscopy technique was applied to measure the DOC concentration in the examined solution. The fact that the DOC fluorescence spectroscopy is not affected by the presence of nitrate in the solution makes it easy to separate the unique effect of absorption by nitrate and DOC. Fluorescence measurements were performed by *TECAN Infinite M200* spectrophotometer with excitation (EX)/Emission (EM) at 350/451nm. The results of the chemical and spectral analyses were used to obtain a series of matrix databases containing nitrate and DOC concentrations and absorbance at 300nm (one per sampling site). In theory, the application of a UV absorption technique on aqueous nitrate solutions should be resulted in a clear linear correlation between the absorption rate and nitrate concentration. Fig. 4 exemplifies said complex pattern in the relationship between nitrate concentration, DOC concentration and the UV absorption for soil water obtained from the conventional greenhouse field station. In fact, since both nitrate and DOC absorb light in different intensities at 300nm, their contribution to the overall absorbance at 300nm can be described as their cumulative absorbance superposition. As such, a 2D model (as presented in Fig. 4) is not so adequate to characterize the relation between N / DOC concentration and the UV absorption. Fig. 4 shows nitrate concentration vs. absorption at 300nm of matrix of water samples obtained from the conventional greenhouse. Each data point represents soil water sample with a known concentration of DOC (indicated by notations next to each point (ppm)) and with known nitrate concentration. It should be noted that the X-axis was converted to a log scale merely to improve the visualization. However, when projecting the data on a 3D domain, it can be seen that the relationship between the three variables creates a plan which can be quantified mathematically (Fig. 5). The applying of a multivariate regression model (e.g., polynomial equation in *MATLAB)* has shown how the nitrate concentration in a sample can be estimated as a function of the DOC and absorbance at 300nm. The following is a general polynomial equation for nitrate estimation:

(2)

$$Nitrate(DOC, Abs) = P_{00} + P_{01}xAbs + P_{10}xDOC$$

Where: Nitrate is the nitrate concentration (ppm), *DOC* indicates the DOC concentration (ppm), *Abs* indicates the absorbance as measured at 300nm (arbitrary units), and $P_{00}$, $P_{10}$, and $P_{01}$ are the coefficients as obtained from the regressions.

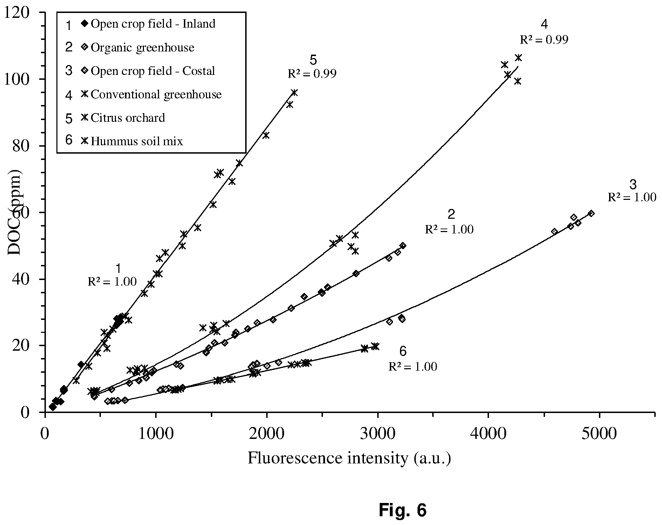

Fluorescence emission at 451nm from samples with known DOC concentrations were used to develop a calibration curve for each site, as shown in Fig. 6. An analysis for the relation between DOC concentration and fluorescence intensity in the various water samples from different fields, respectively, has shown that even though some curves follow a same trend, thus having identical calibration curves (citrus orchard and open field), each group of samples has its own unique calibration equation due to the variation in the DOC chemical and optical characteristics between the various sites. The composition of organic matter that compose the DOC is affected by the site-specific characteristics such as: type of agricultural crop, biological activity in the soil, the type of applied compost, and the type of material used to fertilize. Therefore, calibration curves for DOC concentrations have to be site-specific in order to be properly used in fluorescence spectroscopy. Moreover, since the chemical variability in DOC affects the absorbance spectrum as well, the previously described polynomial equation for estimating nitrate concentration should preferably be site-specific. Nonetheless, it was concluded that the impact of DOC on the absorption spectrum, as resulted from its chemical composition, remains relatively constant over time and is a site-specific feature. This shows that once an initial calibration equation is obtained, this equation can be used for that particular site repeatedly in the long term, making a real-time continuous measurement of nitrate concentration in soil feasible.

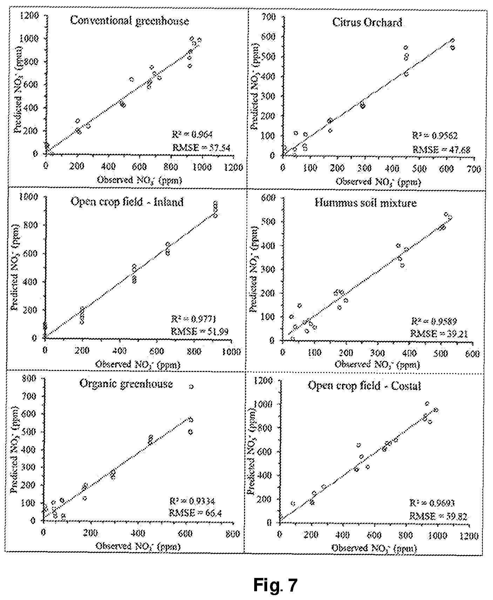

Measurements of DOC concentration, as achieved from fluorescence emission at 451nm, along with the total absorbance at 300nm, enabled the estimation of nitrate concentration in a series of solutions obtained from the previously mentioned field sites (Fig. 7). The concept model successfully estimated nitrate concentrations in all cases, in spite of the presence of DOC in the water samples. The high correlation between the observed and the predicted nitrate measure-

ments (general $R^2 > 0.96$ and average RMSE = 66.4ppm nitrate) shows that the technique is applicable for a continuous, real-time measurement of nitrate concentration in soil as well as in other water sources containing DOC. The method satisfies practical demands for real-time continuous monitoring of nitrate concentration in soil, where the nitrate concentration range may vary from tens to thousands ppm during fertilization and irrigation cycles. Accordingly, LED-based components operating at 280-320nm, and corresponding sensors can be used for the online, *in-situ* nitrate monitoring system in agricultural soils. Nevertheless, the technique of the invention can gain even higher resolution and higher accuracy in the future, when LED UV light sources (212 in Fig. 2) at a wavelength of 200-250nm will be developed and will be commercially available.

In the embodiment above, a calibration with respect to the concentration of nitrate uses a prior knowledge of the type of DOC at the soil where the specific nitrate-concentration determination is actually performed (i.e., what is the additional effect of the specific mixture of the DOC at that area on the absorption). Based on this prior knowledge, a calibration is performed.

In still another embodiment, the identification of the type of DOC at the area may be obtained automatically. In one alternative, the system measures the nitrate concentration in various DOC types by use of a two-phase procedure, as follows:

a. A first phase in which three optical measurements of the solution are performed. The optical measurement may include either two absorption measurements and one fluorescence measurement, or one absorption measurement and two fluorescence measurement. Other combinations of absorbance and fluorescence measurements at many wavelengths may be used to get more accurate determination of the DOC type. Each optical measurement is performed at a different wave length;

b. A second phase in which, based on the results of the above first-phase measurements and prior-knowledge from data-base look-up tables that were prepared for different DOCs in advance, the system identifies which specific calibration equation or calibration curve to use.

In an embodiment of the invention, the analysis unit comprises a mathematical model to extract the value of nitrate based on the measurements of absorption, based on the Beer Lambert equation:

$$A(\lambda) = \varepsilon(\lambda) x C x l$$

Where A is the measured absorbance at a given wavelength ($\lambda$(nm)), $\varepsilon(\lambda)$ is the molar attenuation coefficient (L mol$^{-1}$ cm$^{-1}$) at a given wavelength ($\lambda$(nm)), C is the examined Ion concentration (mol L$^{-1}$) and $l$ is an optical pathlength (cm).

Nitrate concentration can be then obtained by a set of two equations with two unknowns as following:

$$A1(\lambda_1) = A_{NO_3^{-1}}(\lambda_1) + A_{DOC}(\lambda_1) = \varepsilon_{NO_3^{-1}}(\lambda_1) x \left(C_{NO_3^{-1}}\right) x l + \varepsilon_{DOC}(\lambda_1) x (C_{DOC}) x l \ (1)$$

$$A2(\lambda_2) = A_{NO_3^{-1}}(\lambda_2) + A_{DOC}(\lambda_2) = \varepsilon_{NO_3^{-1}}(\lambda_2) x \left(C_{NO_3^{-1}}\right) x l + \varepsilon_{DOC}(\lambda_2) x (C_{DOC}) x l \ (2)$$

From consolidating equation (1)+(2) we can extract the nitrate concentration (equation (3)):

$$C_{NO_3^{-1}} = \frac{C_{DOC}\left(\frac{A_{NO_3^{-1}}(\lambda_1)+A_{DOC}(\lambda_1)}{A_{NO_3^{-1}}(\lambda_2)+A_{DOC}(\lambda_2)}\right) x \varepsilon_{DOC}(\lambda_2) - \varepsilon_{DOC}(\lambda_1)}{\varepsilon_{NO_3^{-1}}(\lambda_1) - \left(\frac{A_{NO_3^{-1}}(\lambda_1)+A_{DOC}(\lambda_1)}{A_{NO_3^{-1}}(\lambda_2)+A_{DOC}(\lambda_2)}\right) x \varepsilon_{NO_3^{-1}}(\lambda_2)} \qquad (3)$$

Where A is the measured absorbance at a given wavelength ($\lambda_{1,2}$(nm)), $\varepsilon_{NO_3^{-1},DOC}\left(\lambda_{1,2}(nm)\right)$ is a molar attenuation coefficient for either the nitrate or the DOC (L mol$^{-1}$ cm$^{-1}$) at a given wavelength ($\lambda_{1,2}$(nm)), $C_{DOC}$ is the DOC concentration (mol L$^{-1}$) as obtained from said second photodetector, $C_{NO3}$ is a nitrate concentration (mol L$^{-1}$), and $l$ is an optical pathlength (cm).

In still another embodiment, a machine learning technique may be used. The machine learning may include the following phases:

a. Generation of many absorption and fluorescence measurements for different values of nitrate concentration and various DOC types and respective concentrations;
b. Selection and adaption of one or more deep learning networks from known results;
c. Training of the network and selection of a one promising a best performance;
d. Use of the trained network to calculate the nitrate concentration based on absorption and fluorescence measurements in a manner as described above.

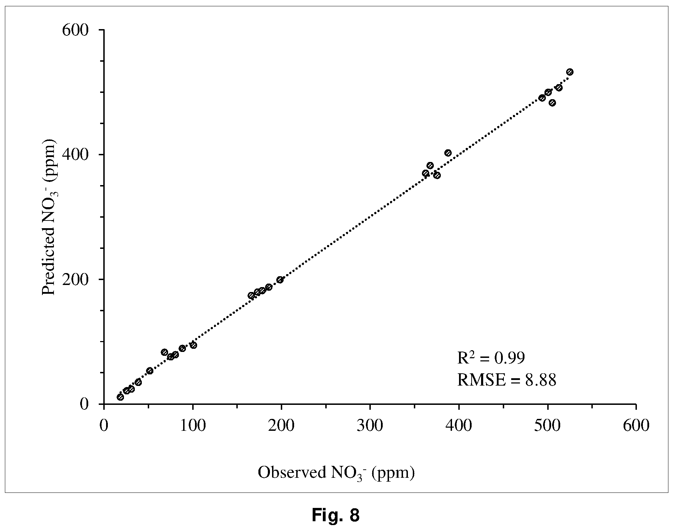

Fig. 8 shows the observed vs. predicted nitrate concentration, for hummus soil mixture water extract, as obtained by the technique of the invention. In this specific experiment the predicted data was obtained for absorbance measurements at 235nm.

While some embodiments of the invention have been described by way of illustration, it will be apparent that the invention can be carried into practice with many modifications, variations and adaptations, and with the use of numerous equivalents or alternative solutions that are within the scope of persons skilled in the art, without departing from or exceeding the scope of the claims.

## Claims

1. A system (200) for determining a level of nitrate (118) in a water sample (112), comprising:

   a. an optical flow cell (206) which is at least partially transparent and which is configured to contain a sample (112) of water;
   b. a first illuminator (212) for illuminating the sample (112) within the cell (206) by light in a first wavelength (312), and a first photodetector (222) for collecting the first-wavelength illumination, following the light passage through the sample (112);

   **characterized by** further comprising:

   c. a second illuminator (214) for illuminating the sample (112) within the cell by light in a second (312), fluorescence-excitation wavelength, and a second photodetector (224) for collecting illumination in a third (324), fluorescence-emission wavelength from the sample; and
   d. an analysis unit for:

      d.1. determining (322) a combined effect of nitrate+Dissolved Organic Carbon, referred to as nitrate+DOC, within the sample (112) on absorbance, said combined effect of nitrate+DOC being proportional to a rate of absorbance of light due to said illumination by said first illuminator (212), said absorbance being determined from a difference between a level of illumination by said first illuminator (212) and a level of collected illumination by said first photodetector (222);
      d.2. given (a) an intensity level by said second illuminator (212) in said second, fluorescence-excitation wavelength (324), (b) a measured emission intensity (254) from the sample in said third, fluorescence-emission wavelength, and (c) a predetermined calibration (340), determining (344) a concentration of DOC within the sample, said DOC concentration being proportional to the intensity (254) of said fluorescence emission from the sample; and
      d.3. deducting said effect of DOC based on the determined DOC concentration by subtracting said effect of DOC from said effect of nitrate+DOC on the absorbance, thereby to determine the concentration of nitrate within the sample.

2. A system according to claim 1, further comprising a first look-up table for converting the difference as measured in step (d. 1) to a nitrate+DOC concentration level.

3. A system according to claim 1, comprising a second look-up table for converting said fluorescence emission as measured in step (d.2) to said DOC concentration level.

4. A system according to claim 1, further comprising a third look-up table, for calibrating the subtraction result of step (d.3) based on a specific type of DOC known to be in the specific tested sample, wherein said type of DOC reflects a specific chemical DOC composition.

5. A system according to claim 1, wherein a mathematical equation is used to convert absorbance and/or fluorescence measurements to concentration levels.

6. A system according to claim 4, further comprising one or more additional illuminators, and one or more additional photodetectors, each measuring absorbance and/or fluorescence emission in additional wavelengths, thereby to determine a specific type of DOC within the sample.

7. A system according to claim 1, wherein said analysis unit comprises a mathematical model (240) to extract the value of nitrate based on said measurements of absorption and fluorescence, wherein the mathematical model comprising:

$$C_{NO_3^{-1}} = \frac{C_{DOC}\left(\frac{A_{NO_3^{-1}}(\lambda_1)+A_{DOC}(\lambda_1)}{A_{NO_3^{-1}}(\lambda_2)+A_{DOC}(\lambda_2)}\right)x\varepsilon_{DOC}(\lambda_2)-\varepsilon_{DOC}(\lambda_1)}{\varepsilon_{NO_3^{-1}}(\lambda_1)-\left(\frac{A_{NO_3^{-1}}(\lambda_1)+A_{DOC}(\lambda_1)}{A_{NO_3^{-1}}(\lambda_2)+A_{DOC}(\lambda_2)}\right)x\varepsilon_{NO_3^{-1}}(\lambda_2)} \qquad (3)$$

Where $A$ is the measured absorbance at a given wavelength ($\lambda_{1,2}$(nm)), $\varepsilon_{NO_3^{-1},DOC}\left(\lambda_{1,2}(nm)\right)$ is a molar attenuation coefficient for either the nitrate or the DOC (L mol$^{-1}$ cm$^{-1}$) at a given wavelength ($\lambda_{1,2}$(nm)), $C_{DOC}$ is the DOC concentration (mol L$^{-1}$) as obtained from said second photodetector, $C_{NO_3}$ is a nitrate concentration (mol L$^{-1}$).

8. A system according to claim 1, wherein said first wavelength is selected from the bands of 200-250nm and 280-320nm, said second excite wavelength is within a band of 225nm-600nm, and said third, fluorescence emission wavelength is within a band of 250nm- 700nm.

9. A system according to claim 1, wherein the water sample (112) is collected from a cultivated soil by a porous interface (202), and is provided in a low flow-rate (204) through the optical flow cell (206).

10. A method for determining a concentration rate (340) of nitrate in a water sample (112), comprising:

    a. providing the sample (112);
    b. illuminating (212) the sample (112) in a first wavelength, and determining (322) a combined effect of nitrate+Dissolved Organic Carbon, referred to as nitrate+DOC within the sample (112) on absorbance, said concentration of nitrate+DOC being proportional to a rate of absorbance of light due to said illumination (212) in said first wavelength, said absorbance being determined from a difference between a level of illumination in said first wavelength before passing the sample(112) and a level of collected illumination (252) in said first wavelength following passage through the water sample (112);

**characterized by** further comprising:

    c. illuminating (214) the water sample (112) in a second, exciting wavelength, and determining (344) the concentration of DOC within the sample (112), said concentration of DOC being proportional to an intensity of fluorescence emission (254) from the sample in a third wavelength due to said illumination (214) of the sample in said second wavelength, wherein said DOC concentration is determined taking into account (a) an intensity level in said second, exciting wavelength, (b) a measured fluorescence emission intensity from the sample (112) in said third wavelength, and (c) a predetermined calibration (232); and
    d. deducting (118) the effect of DOC on the absorbance based on the determined concentration of DOC in the sample by subtracting the effect of DOC from the combined effect (114) of nitrate+DOC on the absorbance, as determined, thereby to obtain the concentration (118) of nitrate in the sample (112).

11. A method according to claim 10, further using a first look-up table for converting said absorbance (252) to a nitrate+DOC concentration levels.

12. A method according to claim 10, using a second look-up table for converting said fluorescence emission to a DOC concentration level.

13. A method according to claim 10, further using a third look-up table, for calibrating the deduction result based on a specific type of DOC known to be in the specific tested sample, wherein said type of DOC reflects a specific chemical DOC composition.

14. A method according to any one of claims 10, wherein a mathematical equation is used to convert absorbance and/or fluorescence measurements to concentration levels.

15. A method according to claim 10, further measuring absorbance and/or fluorescence emission in additional wavelengths, thereby to determine a specific type of DOC within the sample.

16. A method according to claim 10, further comprising use of a mathematical model (240) to extract the value of nitrate based on said measurements of absorption and fluorescence, wherein the mathematical model comprising:

$$C_{NO_3^{-1}} = \frac{C_{DOC}\left(\frac{A_{NO_3^{-1}}(\lambda_1)+A_{DOC}(\lambda_1)}{A_{NO_3^{-1}}(\lambda_2)+A_{DOC}(\lambda_2)}\right)x\varepsilon_{DOC}(\lambda_2)-\varepsilon_{DOC}(\lambda_1)}{\varepsilon_{NO_3^{-1}}(\lambda_1)-\left(\frac{A_{NO_3^{-1}}(\lambda_1)+A_{DOC}(\lambda_1)}{A_{NO_3^{-1}}(\lambda_2)+A_{DOC}(\lambda_2)}\right)x\varepsilon_{NO_3^{-1}}(\lambda_2)} \qquad (3)$$

where $A$ is the measured absorbance at a given wavelength ($\lambda_{1,2}$(nm)), $\varepsilon_{NO_3^{-1},DOC}\left(\lambda_{1,2}(nm)\right)$ is a molar attenuation coefficient for either the nitrate or the DOC (L mol$^{-1}$ cm$^{-1}$) at a given wavelength ($\lambda_{1,2}$(nm)), $C_{DOC}$ is the DOC concentration (mol L$^{-1}$) as obtained from said second photodetector, $C_{NO_3}$ is a nitrate concentration (mol L$^{-1}$).

17. A method according to claim 10, wherein said first wavelength (212) is selected from the bands of 200-250nm, or 280-320nm, wherein said second, excite wavelength (214) is in the order of 225 - 400 nm, and said third, fluorescence emission wavelength is in the order of 250nm-500nm.

18. A method according to claim 10, further applying a machine learning technique, comprising the steps of:

   a. generating a plurality of absorption and fluorescence measurements for different values of nitrate concentration and different types of DOC, and their respective DOC concentrations;
   b. selecting one or more deep learning networks;
   c. training the network and selecting a one with a best performance; and
   d. calculating the nitrate concentration based on said absorption and fluorescence measurements.

**Patentansprüche**

1. System (200) zum Bestimmen eines Nitratgehalts (118) in einer Wasserprobe (112), umfassend:

   a. eine optische Strömungszelle (206), die mindestens teilweise transparent ist und dazu konfiguriert ist, eine Probe (112) von Wasser aufzunehmen;
   b. einen ersten Beleuchtungskörper (212) zum Beleuchten der Probe (112) innerhalb der Zelle (206) mit Licht in einer ersten Wellenlänge (312), und einen ersten Fotodetektor (222) zum Auffangen der Beleuchtung mit der ersten Wellenlänge nachdem das Licht die Probe (112) durchlaufen hat;

   **dadurch gekennzeichnet, dass** es ferner umfasst:

   c. einen zweiten Beleuchtungskörper (214) zum Beleuchten der Probe (112) innerhalb der Zelle mit Licht einer zweiten (312) Fluoreszenzanregungswellenlänge und einen zweiten Fotodetektor (224) zum Auffangen der Beleuchtung in einer dritten (324) Fluoreszenzemissionswellenlänge von der Probe; und
   d. eine Analyseeinheit zum:

   d.1. Bestimmen (322) einer kombinierten Wirkung von Nitrat + gelöstem organischem Kohlenstoff, bezeichnet als Nitrat+DOC, innerhalb der Probe (112) auf die Absorption, wobei die kombinierte Wirkung von Nitrat+DOC proportional zu einer Absorbanzrate von Licht aufgrund der Beleuchtung durch den ersten Beleuchtungskörper (212) ist, und die Absorption bestimmt wird aus einer Differenz zwischen einem Beleuchtungspegel durch den ersten Beleuchtungskörper (212) und einem Pegel der gesammelten Beleuchtung durch den ersten Fotodetektor (222);

d.2. bei (a) einem Intensitätspegel des zweiten Beleuchtungskörpers (212) in der zweiten Fluoreszenzanregungswellenlänge (324), (b) einer gemessenen Emissionsintensität (254) von der Probe in der dritten Fluoreszenzemissionswellenlänge und (c) einer vorgegebenen Kalibrierung (340), Bestimmen (344) einer DOC-Konzentration in der Probe, wobei die DOC-Konzentration proportional ist zu der Intensität (254) der Fluoreszenzemission von der Probe; und

d.3. Ableiten der Wirkung von DOC auf der Grundlage der bestimmten DOC-Konzentration durch Subtrahieren der Wirkung von DOC von der Wirkung von Nitrat+DOC auf die Absorbanz, um dadurch die Nitratkonzentration in der Probe zu bestimmen.

2. System nach Anspruch 1, ferner umfassend eine erste Nachschlagetabelle zum Umwandeln der in Schritt (d.1) gemessenen Differenz in einen Nitrat+DOC-Konzentrationspegel.

3. System nach Anspruch 1, umfassend eine zweite Nachschlagetabelle zum Umwandeln der in Schritt (d.2) gemessenen Fluoreszenzemission in den DOC-Konzentrationspegel.

4. System nach Anspruch 1, ferner umfassend eine dritte Nachschlagetabelle zum Kalibrieren des Subtraktionsergebnisses aus Schritt (d.3) auf der Grundlage eines bestimmten DOC-Typs, von dem bekannt ist, dass er in der bestimmten getesteten Probe vorliegt, wobei dieser DOC-Typ eine bestimmte chemische DOC-Zusammensetzung widerspiegelt.

5. System nach Anspruch 1, bei dem eine mathematische Gleichung verwendet wird, um Absorbanz- und/oder Fluoreszenzmessungen in Konzentrationspegel umzuwandeln.

6. System nach Anspruch 4, ferner umfassend einen oder mehrere zusätzliche Beleuchtungskörper und einen oder mehrere zusätzliche Fotodetektoren, die jeweils die Absorbanz und/oder Fluoreszenzemission in zusätzlichen Wellenlängen messen, um dadurch einen bestimmten DOC-Typ innerhalb der Probe zu bestimmen.

7. System nach Anspruch 1, wobei die Analyseeinheit ein mathematisches Modell (240) umfasst, um den Nitratwert auf der Grundlage der Absorptions- und Fluoreszenzmessungen zu extrahieren, das mathematische Modell umfassend:

$$C_{NO_3^{-1}} = \frac{C_{DOC}\left(\frac{A_{NO_3^{-1}}(\lambda_1)+A_{DOC}(\lambda_1)}{A_{NO_3^{-1}}(\lambda_2)+A_{DOC}(\lambda_2)}\right)x\varepsilon_{DOC}(\lambda_2)-\varepsilon_{DOC}(\lambda_1)}{\varepsilon_{NO_3^{-1}}(\lambda_1)-\left(\frac{A_{NO_3^{-1}}(\lambda_1)+A_{DOC}(\lambda_1)}{A_{NO_3^{-1}}(\lambda_2)+A_{DOC}(\lambda_2)}\right)x\varepsilon_{NO_3^{-1}}(\lambda_2)} \qquad (3)$$

wobei A die gemessene Absorbanz bei einer bestimmten Wellenlänge ($\lambda_{1,2}$(nm)) ist, $\varepsilon_{NO_3^{-1},\,DOC}(\lambda_{1,2}(nm))$ ein molarer Dämpfungskoeffizient für entweder das Nitrat oder für DOC (L mol$^{-1}$cm$^{-1}$) bei einer bestimmten Wellenlänge ($\lambda_{1,2}$(nm)) ist, $C_{DOC}$ die DOC-Konzentration (mol L$^{-1}$) ist, wie von dem zweiten Fotodetektor erhalten, und $C_{NO_3}$ eine Nitratkonzentration (mol L$^{-1}$) ist.

8. System nach Anspruch 1, wobei die erste Wellenlänge ausgewählt ist aus den Bändern 200-250 nm und 280-320 nm, die zweite Anregungswellenlänge innerhalb eines Bandes von 225-600 nm liegt und die dritte Fluoreszenzemissionswellenlänge innerhalb eines Bandes von 250-700 nm liegt.

9. System nach Anspruch 1, wobei die Wasserprobe (112) aus einem kultivierten Boden durch eine poröse Schnittstelle (202) gesammelt und mit einer niedrigen Strömungsrate (204) durch die optische Strömungszelle (206) bereitgestellt wird.

10. Verfahren zum Bestimmen einer Konzentrationsrate (340) von Nitrat in einer Wasserprobe (112), umfassend:

a. Bereitstellen der Probe (112);

b. Beleuchten (212) der Probe (112) mit einer ersten Wellenlänge und Bestimmen (322) einer kombinierten Wirkung von Nitrat + gelöstem organischem Kohlenstoff, bezeichnet als Nitrat+DOC in der Probe (112), auf die Absorbanz, wobei die Konzentration von Nitrat+DOC proportional zu einer Absorbanzrate von Licht aufgrund der Beleuchtung (212) mit der ersten Wellenlänge ist und die Absorbanz aus einer Differenz zwischen einem Beleuchtungspegel mit der ersten Wellenlänge vor dem Durchlaufen der Probe (112) und einem Pegel der gesammelten Beleuchtung (252) mit der ersten Wellenlänge nach dem Durchlaufen der Wasserprobe (112) bestimmt wird; **dadurch gekennzeichnet, dass** es ferner umfasst:

c. Beleuchten (214) der Wasserprobe (112) mit einer zweiten, Anregungswellenlänge und Bestimmen (344) der DOC-Konzentration in der Probe (112), wobei die DOC-Konzentration proportional zu einer Intensität der Fluoreszenzemission (254) von der Probe bei einer dritten Wellenlänge aufgrund der Beleuchtung (214) der Probe bei der zweiten Wellenlänge ist, wobei die DOC-Konzentration unter Berücksichtigung (a) eines Intensitätspegels bei der zweiten, Anregungswellenlänge, (b) einer gemessenen Fluoreszenzemissionsintensität der Probe (112) bei der dritten Wellenlänge und (c) einer vorgegebenen Kalibrierung (232) bestimmt wird; und
d. Ableiten (118) der Wirkung von DOC auf die Absorbanz basierend auf der bestimmten DOC-Konzentration in der Probe durch Subtrahieren der Wirkung von DOC von der kombinierten Wirkung (114) von Nitrat+DOC auf die Absorbanz, wie bestimmt, um dadurch die Konzentration (118) von Nitrat in der Probe (112) zu erhalten.

11. Verfahren nach Anspruch 10, bei dem ferner eine erste Nachschlagetabelle zum Umwandeln der Absorbanz (252) in Nitrat+DOC-Konzentrationspegel verwendet wird.

12. Verfahren nach Anspruch 10, bei dem eine zweite Nachschlagetabelle zum Umwandeln der Fluoreszenzemission in einen DOC-Konzentrationspegel verwendet wird.

13. Verfahren nach Anspruch 10, bei dem ferner eine dritte Nachschlagetabelle zum Kalibrieren des Ableitungsergebnisses aus auf der Grundlage eines bestimmten DOC-Typs, von dem bekannt ist, dass er in der bestimmten getesteten Probe vorliegt, wobei dieser DOC-Typ eine bestimmte chemische DOC-Zusammensetzung widerspiegelt.

14. Verfahren nach einem der Ansprüche 10, bei dem eine mathematische Gleichung verwendet wird, um Absorbanz- und/oder Fluoreszenzmessungen in Konzentrationspegel umzuwandeln.

15. Verfahren nach Anspruch 10, wobei ferner Absorbanz und/oder Fluoreszenzemission in zusätzlichen Wellenlängen gemessen wird, um dadurch einen bestimmten DOC-Typ innerhalb der Probe zu bestimmen.

16. Verfahren nach Anspruch 10, ferner umfassend die Verwendung eines mathematischen Modells (240), um den Nitratwert auf der Grundlage der Absorptions- und Fluoreszenzmessungen zu extrahieren, das mathematische Modell umfassend:

$$C_{NO_3^{-1}} = \frac{C_{DOC}\left(\frac{A_{NO_3^{-1}}(\lambda_1)+A_{DOC}(\lambda_1)}{A_{NO_3^{-1}}(\lambda_2)+A_{DOC}(\lambda_2)}\right)x\varepsilon_{DOC}(\lambda_2)-\varepsilon_{DOC}(\lambda_1)}{\varepsilon_{NO_3^{-1}}(\lambda_1)-\left(\frac{A_{NO_3^{-1}}(\lambda_1)+A_{DOC}(\lambda_1)}{A_{NO_3^{-1}}(\lambda_2)+A_{DOC}(\lambda_2)}\right)x\varepsilon_{NO_3^{-1}}(\lambda_2)} \qquad (3)$$

wobei A die gemessene Absorbanz bei einer bestimmten Wellenlänge ($\lambda_{1,2}$(nm)) ist, $\varepsilon_{NO_3^{-1}, DOC}(\lambda_{1,2}(nm))$ ein molarer Dämpfungskoeffizient für entweder das Nitrat oder für DOC (L mol$^{-1}$cm$^{-1}$) bei einer bestimmten Wellen-länge ($\lambda_{1,2}$(nm)) ist, $C_{DOC}$ die DOC-Konzentration (mol L$^{-1}$) ist, wie von dem zweiten Fotodetektor erhalten, und $C_{NO_3}$ eine Nitratkonzentration (mol L$^{-1}$) ist.

17. Verfahren nach Anspruch 10, wobei die erste Wellenlänge (212) ausgewählt ist aus den Bändern 200-250 nm und 280-320 nm, die zweite Anregungswellenlänge (214) im Bereich 225-400 nm liegt und die dritte Fluoreszenzemissionswellenlänge im Bereich 250-500 nm liegt.

18. Verfahren nach Anspruch 10, das ferner eine Maschinenlerntechnik anwendet, umfassend die Schritte:

a. Erzeugen einer Vielzahl von Absorptions- und Fluoreszenzmessungen für unterschiedliche Nitratkonzentrationswerte und unterschiedliche DOC-Typen sowie für ihre jeweiligen DOC-Konzentrationen;
b. Auswählen eines oder mehrerer Deep-Learning-Netzwerke;
c. Trainieren des Netzwerks und Auswählen des Netzwerks mit der besten Leistung; und
d. Berechnen der Nitratkonzentration auf der Grundlage der Absorptions- und Fluoreszenzmessungen.

**Revendications**

1. Système (200) pour déterminer un niveau de nitrate (118) dans un échantillon d'eau (112), comprenant :

   a. une cellule à flux optique (206) qui est au moins partiellement transparente et qui est configurée pour contenir un échantillon (112) d'eau ;
   b. un premier illuminateur (212) pour illuminer l'échantillon (112) à l'intérieur de la cellule (206), par le biais de lumière dans une première longueur d'onde (312), et un premier photodétecteur (222) pour collecter l'illumination en première longueur d'onde, suivant le passage de lumière à travers l'échantillon (112) ;

   **caractérisé en ce qu'**il comprend en outre :

   c. un second illuminateur (214) pour illuminer l'échantillon (112) à l'intérieur de la cellule, par le biais de lumière dans une deuxième longueur d'onde (312) d'excitation de fluorescence, et un second photodétecteur (224) pour collecter l'illumination dans une troisième longueur d'onde (324) d'émission de fluorescence provenant de l'échantillon ; et
   d. une unité d'analyse pour :

      d.1. déterminer (322) un effet combiné de nitrate + carbone organique dissout (« Dissolved Organic Carbon »), appelé nitrate + DOC, à l'intérieur de l'échantillon (112), sur l'absorbance, ledit effet combiné de nitrate + DOC étant proportionnel à un taux d'absorbance de lumière dû à ladite illumination par ledit premier illuminateur (212), ladite absorbance étant déterminée à partir d'une différence entre un niveau d'illumination par ledit premier illuminateur (212) et un niveau d'illumination collectée par ledit premier photodétecteur (222) ;
      d.2. étant donnés (a) un niveau d'intensité, par ledit second illuminateur (212), dans ladite deuxième longueur d'onde (324) d'excitation de fluorescence, (b) une intensité d'émission mesurée (254), provenant de l'échantillon, dans ladite troisième longueur d'onde d'émission de fluorescence, et (c) un étalonnage prédéterminé (340), déterminer (344) une concentration de DOC à l'intérieur de l'échantillon, ladite concentration de DOC étant proportionnelle à l'intensité (254) de ladite émission de fluorescence provenant de l'échantillon ; et
      d.3. déduire ledit effet de DOC, sur la base de la concentration de DOC déterminée, en soustrayant ledit effet de DOC dudit effet de nitrate + DOC sur l'absorbance, pour ainsi déterminer la concentration de nitrate à l'intérieur de l'échantillon.

2. Système selon la revendication 1, comprenant en outre une première table de consultation pour convertir la différence telle que mesurée dans ladite étape (d.1) en un niveau de concentration de nitrate + DOC.

3. Système selon la revendication 1, comprenant une deuxième table de consultation pour convertir ladite émission de fluorescence telle que mesurée dans ladite étape (d.2) en ledit niveau de concentration de DOC.

4. Système selon la revendication 1, comprenant en outre une troisième table de consultation, pour étalonner le résultat de soustraction de l'étape (d.3), sur la base d'un type spécifique de DOC, dont l'existence dans l'échantillon testé spécifique est connue, dans lequel ledit type de DOC reflète une composition de DOC chimique spécifique.

5. Système selon la revendication 1, dans lequel une équation mathématique est utilisée pour convertir des mesures d'absorbance et/ou de fluorescence en niveaux de concentration.

6. Système selon la revendication 4, comprenant en outre un ou plusieurs illuminateurs supplémentaires, et un ou plusieurs photodétecteurs supplémentaires, chacun mesurant l'absorbance et/ou l'émission de fluorescence dans des longueurs d'onde supplémentaires, pour ainsi déterminer un type spécifique de DOC à l'intérieur de l'échantillon.

7.  Système selon la revendication 1, dans lequel ladite unité d'analyse comprend un modèle mathématique (240) pour extraire la valeur de nitrate sur la base desdites mesures d'absorption et de fluorescence, dans lequel le modèle mathématique comprend :

$$C_{NO_3^{-1}} = \frac{C_{DOC}\left(\frac{A_{NO_3^{-1}}(\lambda_1)+A_{DOC}(\lambda_1)}{A_{NO_3^{-1}}(\lambda_2)+A_{DOC}(\lambda_2)}\right)x\varepsilon_{DOC}(\lambda_2)-\varepsilon_{DOC}(\lambda_1)}{\varepsilon_{NO_3^{-1}}(\lambda_1)-\left(\frac{A_{NO_3^{-1}}(\lambda_1)+A_{DOC}(\lambda_1)}{A_{NO_3^{-1}}(\lambda_2)+A_{DOC}(\lambda_2)}\right)x\varepsilon_{NO_3^{-1}}(\lambda_2)} \qquad (3)$$

où $A$ est l'absorbance mesurée à une longueur d'onde donnée ($\lambda_{1,2}$(nm)), $\varepsilon_{NO_3^{-1},DOC}\left(\lambda_{1,2}(nm)\right)$ est un coefficient d'atténuation molaire pour le nitrate ou pour le DOC (L mol$^{-1}$ cm$^{-1}$) à une longueur d'onde donnée ($\lambda_{1,2}$ (nm)), $C_{DOC}$ est la concentration de DOC (mol L$^{-1}$) telle qu'obtenue dudit second photodétecteur, $C_{NO_3}$ est une concentration de nitrate (mol L$^{-1}$).

8.  Système selon la revendication 1, dans lequel ladite première longueur d'onde est sélectionnée parmi les bandes de 200-250 nm et 280-320 nm, ladite deuxième longueur d'onde d'excitation est dans les limites d'une bande de 225 nm-600 nm, et ladite troisième longueur d'onde d'émission de fluorescence est dans les limites d'une bande de 250 nm-700 nm.

9.  Système selon la revendication 1, dans lequel l'échantillon d'eau (112) est prélevé à partir d'un sol cultivé par une interface poreuse (202), et est fourni en un bas débit (204) à travers la cellule à flux optique (206).

10. Procédé pour déterminer un taux de concentration (340) de nitrate dans un échantillon d'eau (112), comprenant :

    a. fournir l'échantillon (112) ;
    b. illuminer (212) l'échantillon (112), dans une première longueur d'onde, et déterminer (322) un effet combiné de nitrate + carbone organique dissout (« Dissolved Organic Carbon »), appelé nitrate + DOC, à l'intérieur de l'échantillon (112), sur l'absorbance, ladite concentration de nitrate + DOC étant proportionnelle à un taux d'absorbance de lumière dû à ladite illumination (212) dans ladite première longueur d'onde, ladite absorbance étant déterminée à partir d'une différence entre un niveau d'illumination dans ladite première longueur d'onde avant le passage à travers l'échantillon (112) et un niveau d'illumination collectée (252) dans ladite première longueur d'onde suivant le passage à travers l'échantillon d'eau (112), **caractérisé en ce qu'**il comprend en outre :

    c. illuminer (214) l'échantillon d'eau (112) dans une deuxième longueur d'onde d'excitation, et déterminer (344) la concentration de DOC à l'intérieur de l'échantillon (112), ladite concentration de DOC étant proportionnelle à une intensité d'émission de fluorescence (254) provenant de l'échantillon, dans une troisième longueur d'onde, due à ladite illumination (214) de l'échantillon dans ladite deuxième longueur d'onde, dans lequel ladite concentration de DOC est déterminée en prenant en compte (a) un niveau d'intensité dans ladite deuxième longueur d'onde d'excitation, (b) une intensité d'émission fluorescence mesurée provenant de l'échantillon (112) dans ladite troisième longueur d'onde, et (c) un étalonnage prédéterminé (232) ; et
    d. déduire (118) l'effet de DOC sur l'absorbance, sur la base de la concentration de DOC déterminée dans l'échantillon, en soustrayant l'effet de DOC de l'effet combiné (114) de nitrate + DOC sur l'absorbance, tel que déterminé, pour ainsi obtenir la concentration (118) de nitrate dans l'échantillon (112).

11. Procédé selon la revendication 10, utilisant en outre une première table de consultation pour convertir ladite absorbance (252) en un niveau de concentration de nitrate + DOC.

12. Procédé selon la revendication 10, utilisant une deuxième table de consultation pour convertir ladite émission de fluorescence en un niveau de concentration de DOC.

13. Procédé selon la revendication 10, utilisant en outre une troisième table de consultation, pour étalonner le résultat de déduction, sur la base d'un type spécifique de DOC, dont l'existence dans l'échantillon testé spécifique est connue, dans lequel ledit type de DOC reflète une composition de DOC chimique spécifique.

**14.** Procédé selon l'une quelconque des revendications 10, dans lequel une équation mathématique est utilisée pour convertir des mesures d'absorbance et/ou de fluorescence en niveaux de concentration.

**15.** Procédé selon la revendication 10, mesurant en outre l'absorbance et/ou l'émission de fluorescence dans des longueurs d'onde supplémentaires, pour ainsi déterminer un type spécifique de DOC à l'intérieur de l'échantillon.

**16.** Procédé selon la revendication 10, comprenant en outre l'utilisation d'un modèle mathématique (240) pour extraire la valeur de nitrate, sur la base desdites mesures d'absorption et de fluorescence, dans lequel le modèle mathématique comprend :

$$C_{NO_3^{-1}} = \frac{C_{DOC}\left(\frac{A_{NO_3^{-1}}(\lambda_1)+A_{DOC}(\lambda_1)}{A_{NO_3^{-1}}(\lambda_2)+A_{DOC}(\lambda_2)}\right)x\varepsilon_{DOC}(\lambda_2)-\varepsilon_{DOC}(\lambda_1)}{\varepsilon_{NO_3^{-1}}(\lambda_1)-\left(\frac{A_{NO_3^{-1}}(\lambda_1)+A_{DOC}(\lambda_1)}{A_{NO_3^{-1}}(\lambda_2)+A_{DOC}(\lambda_2)}\right)x\varepsilon_{NO_3^{-1}}(\lambda_2)} \qquad (3)$$

où $A$ est l'absorbance mesurée à une longueur d'onde donnée ($\lambda_{1,2}$(nm)), $\varepsilon_{NO_3^{-1},DOC}\left(\lambda_{1,2}(nm)\right)$ est un coefficient d'atténuation molaire pour le nitrate ou pour le DOC (L mol$^{-1}$ cm$^{-1}$) à une longueur d'onde donnée ($\lambda_{1,2}$ (nm)), $C_{DOC}$ est la concentration de DOC (mol L$^{-1}$) telle qu'obtenue dudit second photodétecteur, $C_{NO_3}$ est une concentration de nitrate (mol L$^{-1}$).

**17.** Procédé selon la revendication 10, dans lequel ladite première longueur d'onde (212) est sélectionnée parmi les bandes de 200-250 nm, ou de 280-320 nm, dans lequel ladite deuxième longueur d'onde d'excitation (214) est dans l'ordre de 225-400 nm, et ladite troisième longueur d'onde d'émission de fluorescence est dans l'ordre de 250 nm-500 nm.

**18.** Procédé selon la revendication 10, appliquant en outre une technique d'apprentissage machine, comprenant les étapes consistant à :

a. générer une pluralité de mesures d'absorption et de fluorescence pour différentes valeurs de concentration de nitrate et différents types de DOC, et leurs concentrations de DOC respectives ;
b. sélectionner un ou plusieurs réseaux d'apprentissage profond ;
c. entraîner le réseau et sélectionner un avec une meilleure performance ; et
d. calculer la concentration de nitrate sur la base desdites mesures d'absorption et de fluorescence.

100

112

Obtaining a water sample

114

Illuminating the sample and
determining the combined effect of
N+DOC on the light absorbance

116

Illuminating the sample and
determining the concentration of DOC
based on fluorescence emission

118

Determining the concentration
of N by subtucting the effect of
DOC from the combined effect
of N+DOC

**Fig. 1**

Fig. 2

300
314

312

```
┌─────────────────────┐                              ┌─────────────────────┐
│  Illuminating in a 2ⁿᵈ │                              │  Illuminating in a 1ˢᵗ  │
│     wavelength      │                              │     wavelength      │
└─────────────────────┘                              └─────────────────────┘
```

324

322

```
┌─────────────────────┐                              ┌─────────────────────┐
│  Detecting illumination │                           │ Determining total absorbance │
│ (fluorescence emission) │                           │      due to N+DOC      │
│   in a 3ʳᵈ wavelength  │                            └─────────────────────┘
└─────────────────────┘
```

364                                                  332

```
┌─────────────────────┐                              ┌─────────────────────┐
│   Calculate using a   │                             │  Calculate using a 1ˢᵗ │
│      2ⁿᵈ L.U.T       │                              │        L.U.T        │
└─────────────────────┘                              └─────────────────────┘
```

344                                                                          340

```
┌─────────────────────┐                              ┌─────────────────────────┐
│     Determining      │                             │    Polynomial Cal.     │
│  concentration rate of │ ──────────────────────────▶│  N concentration as a   │
│         DOC          │                             │ function of Total absorbance │
└─────────────────────┘                              │  and DOC concertation  │
                                                      └─────────────────────────┘
```

**Fig. 3**

**Fig. 4**

EP 3 963 308 B1

Fig. 5

Fig. 6

Fig. 7

24

**Fig. 8**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018104939 A1, Yeshno
- CN 109655110 A
- WO 2017156258 A1

**Non-patent literature cited in the description**

- **TULY et al.** *In Situ Monitoring of Soil Solution Nitrate: Proof of Concept*, 2009, https://www.researchgate.net/publication/231523625
- **B.D. SHAW et al.** Analysis of Ion and Dissolved Organic Carbon Interference on Soil Solution Nitrate Concentration Measurements Using Ultraviolet Absorption Spectroscopy. *VADOSE ZONE JOURNAL*, 19 December 2014, vol. 13 (12), 1-9
- **ANTHONY C. EDWARDS et al.** Determination of Nitrate in Water Containing Dissolved Organic Carbon by Ultraviolet Spectroscopy. *International Journal of Environmental Analytical Chemistry*, 2001, vol. 80 (1), 49-59